(19) Europäisches Patentamt
European Patent Office
Office européen des brevets

(11) Publication number: 0 572 365 A2

# (12) EUROPEAN PATENT APPLICATION

(21) Application number: **93830237.9**

(22) Date of filing: **28.05.93**

(51) Int. Cl.[5]: **C07D 207/28**, C07D 207/16

(30) Priority: **29.05.92 IT RM920412**

(43) Date of publication of application: **01.12.93 Bulletin 93/48**

(84) Designated Contracting States: **AT BE CH DE DK ES FR GB GR IE IT LI LU MC NL PT SE**

(71) Applicant: **NUOVO CONSORZIO SANITARIO NAZIONALE del Dott. Paolo Malizia**
**Via Svetonio 6**
**I-00136 Roma(IT)**

(72) Inventor: **Casini, Giovanni**
**379 Via Camerata Picena**
**I-00136 Roma(IT)**

(74) Representative: **Bazzichelli, Alfredo et al**
**c/o Società Italiana Brevetti S.p.A.**
**Piazza di Pietra, 39**
**I-00186 Roma (IT)**

## (54) Heteroprostanoids, process for their production and therapeutical use thereof.

(57) The present invention relates to new heteroprostanoids of general formula IV

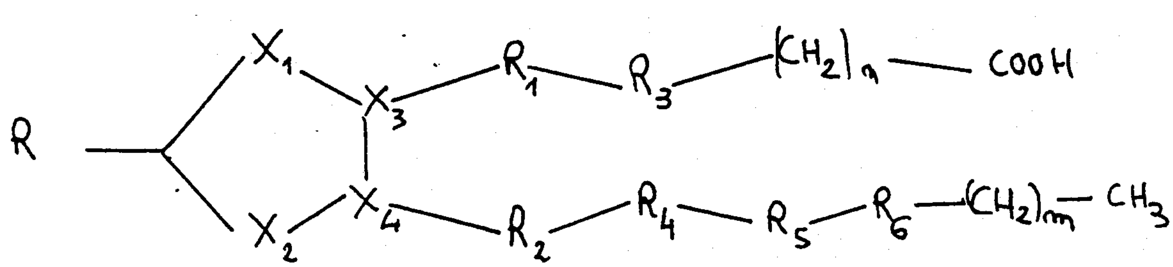

in which is $X_1 = CH_2$, CO; $X_2 = CH_2$, CO; $X_3$ = N, CH; $X_4$ = N, CH; R = H, OH; $R_1 = CH_2$, CO; $R_2$ = CO, $CH_2$; $R_3 = CH_2$, NH, O; $R_4$ = NH, $CH_2$, CO; $R_5 = CH_2$, NH; $R_6 = CH_2$, CO; m is comprised between 0 and 4; n is comprised between 0 and 5, and to a process for their production.

The use of same compounds as active agents on smooth muscles and against thrombocytes aggregation and pharmaceutical compositions containing same compounds are also described.

EP 0 572 365 A2

The present invention relates to heteroprostanoids, a process for their production and their therapeutical use.

Natural prostaglandins (PG) are a class of natural substances, endogenous in the human and animal organisms, which were discovered around 1960 by Bergstrom et al. Prostaglandins derive metabolically from arachidonic acid, which is thus their biochemical precursor, by means of an enzymatic system called prostaglandin-synthetase (PG-synthetase). These compounds are produced by different organs or tissues in the organism, and they have a number of functions for regulation of cell activity: they stimulate vasal smooth muscles, intestinal muscles and that of other organs, for example the uterus; they protect the gastric mucosa against aggression by gastric juices; they regulate a number of metabolic activities; they intervene in inflammatory processes; they regulate thrombocyte aggregation, etc.

From a structural point of view they are all derived from the common skeleton of trans-2-n-octyl-cyclopentan-1-heptanoic acid (prostanoic acid) of general formula V

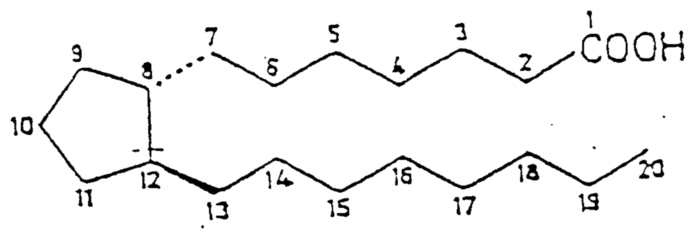

and contain various insaturations in the carbonious skeleton of this acid ( △ -13,14-trans in all prostaglandins, △ -5,6-cis in PG2 and PG3, △ -17, 18-cis in PG3 only), along with several functional groups (15-α-OH in all PGs, 9-α-OH in PGD and PGF-α, 11-α-OH in PGE and PGF-α, 9=0 in pGE, 11=0 in PGD). The various prostaglandins have been indicated following the official nomenclature. In literature, as well as these compounds, more complex derivatives are also described having an oxygenated heterocycle, such as PGI (prostacyclin). Natural prostaglandins are used in therapy, especially in obstetrics, to facilitate labour or dilation of the cervix (PGF2-α, known by the international non proprietory name of Dinoprost, and PGE2, known by the common international non proprietory name of Dinoprostone), or in heart surgery (PGE1, known by the international non proprietory name of Alprostadil). However, natural prostaglandins are characterized by high chemical and metabolic lability, consequently their action is of extremely short duration; for this reason their use in other therapeutic fields is very limited.

Other similar, synthetically produced compounds also belong to the state of the art. These are more stable, and thus have a longer action and are also used in obstetrics (international non proprietory name Carboprost) or as gastroprotectors (international non proprietory names Misoprostol and Rosaprostol). However, the above mentioned compounds have structures which are difficult to synthesize and which also have the disadvantage of being completely foreign to the organism due to the structural modifications introduced by synthesis.

More easily synthesized structures also form part of the state of the art, for example those containing heteroatoms introduced into the cyclopentanic mojety of prostanoic acid (heteroprostanoids). It is therefore possible to trace back to this class of compounds, called heteroprostanoids, certain compounds described in the literature and called aza- and oxaprostanoids, and certain diaza- and triazaprostanoids starting from simple heterocycles such as hydantoins or triazolidindiones. Although certain of these compounds have shown prostaglandin- like activity, only one of them has been introduced in therapy for obstetric use (known as Sulprostone). However, these compounds can still be considered as entirely foreign to the organism, and therefore potentially extremely toxic both as such and, above all, due to any metabolites they may produce (normally indicated as hard drugs).

It has now surprisingly been discovered that it is possible to obtain through a synthetic route heteroprostanoid structures which, as well as having prostaglandin-like activity comparable with that of natural prostaglandins and their synthetic analogs known from the state of the art, do not show the chemical lability of natural prostaglandins and therefore have longer lasting action, and furthermore show low toxicity as such and, above all, in the metabolytes they produce, which are non-toxic natural metabolites (so-called soft drugs).

Object of the present invention are therefore heteroprostanoids and their homologs of general formula IV

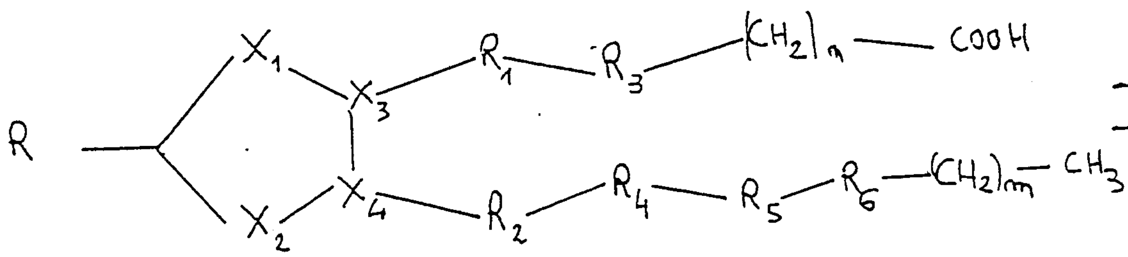

IV

in which is $X_1 = CH_2$, CO; $X_2 = CH_2$, CO; $X_3 = N$, CH; $X_4 = N$, CH; R = H, OH; $R_1 = CH_2$, CO; $R_2 = CO$, $CH_2$; $R_3 = CH_2$, NH, O; $R_4 = NH$, $CH_2$, CO; $R_5 = CH_2$, NH; $R_6 = CH_2$, CO; m between 0 and 4; $Y = (CH_2)_n$ with n between 0 and 5.

In particular, the present invention relates to compounds of formula I derived from those of general formula IV, in which

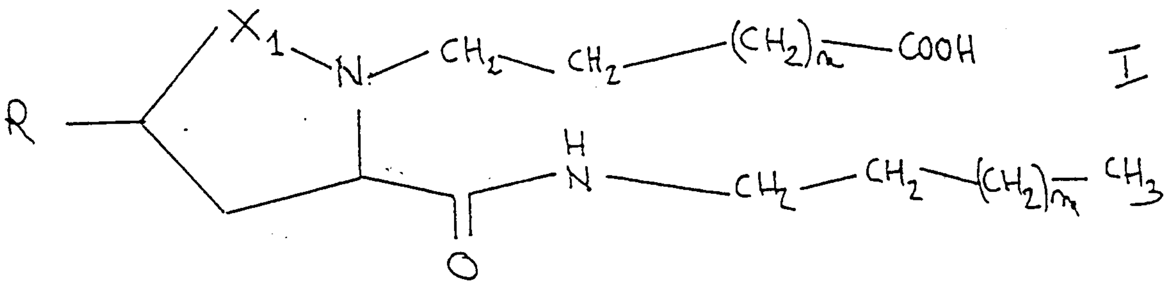

I

$X_2 = CH_2$; $X_3 = N$; $X_4 = CH$; $R_1 = CH_2$; $R_2 = CO$; $R_3 = CH_2$;$R_4 = NH$; $R_5 = CH_2$; $R_6 = CH_2$, which can be generally defined as 8,14-diazaprostanoic acids.

In particular the present invention also relates to compounds of formula II derived from those of general formula IV and in which $X_1 = CH_2$; $X_3 = CH$; $X_4 = N$; $R_1 = CO$; $R_3 = NH$; $R_5 = NH$;

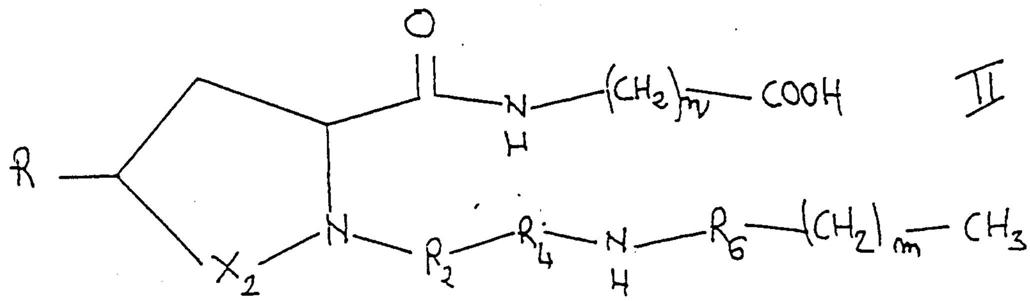

II

which can be generally defined as 6,12,15-triazaprostanoic acids.

In particular the present invention also relates to compounds of general formula III derived from general formula IV, in which

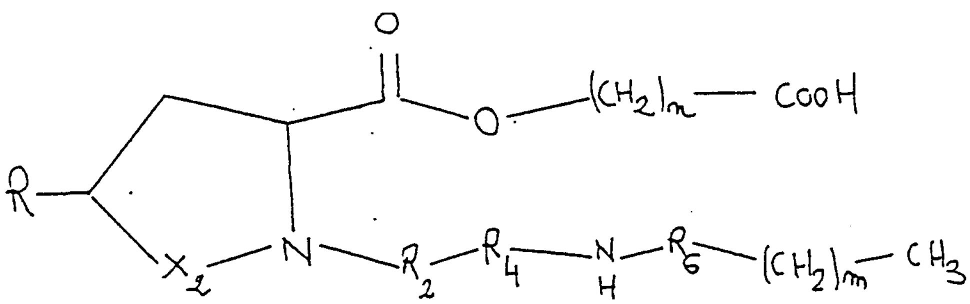

III

$X_1 = CH_2$; $X_3 = CH$; $X_4 = N$; $R_1 = CO$; $R_3 = O$; $R_5 = NH$; which can be defined as 12-15-diaza-6-oxa-prostanoic acids.

A further object of the present invention is the therapeutic use of the above compounds for control of smooth muscle activity and to inhibit thrombocyte aggregation.

A further object of the present invention are pharmaceutical formulations containing therapeutically effective amounts of at least one compound according to the present invention along with usual pharmaceutical excipients and vehicles.

The compounds according to the present invention can be obtained according to a general pattern (G), which also forms an object of the present invention, consisting in three successive steps at the end of which compounds of general formula XI are formed, which can be traced to those of general formula IV:

1) a carboxylic compound of general formula VI, in which R = H, OBz and R' = H, BOC is transformed into a functional derivative thereof to the carboxylic function, of general formula VIII, by reacting it with a compound of general formula VII in which A' = halogen, NHBz or $NH_2$; J = $CH_3$, COOH, the COOH group being optionally protected, $0 \leqq r \leqq 7$, $X_2$ having the values indicated in the text;

2) the compound of general formula VIII obtained from step 1, in which A = O, NH or NBz is reacted (if necessary after deprotection of the R' group) with a compound of formula IX in which D is an halogen or an OH, and Z is a methylenic chain with up to 8 members, optionally interrupted by CO and/or NH groups;

3) the compound of general formula X, obtained from step 2, is then deprotected by catalytic hydrogenization so as to free any protected functions and to give the compound of formula XI.

The order of steps 1 and 2 can also be inverted.

**DIAGRAM G**

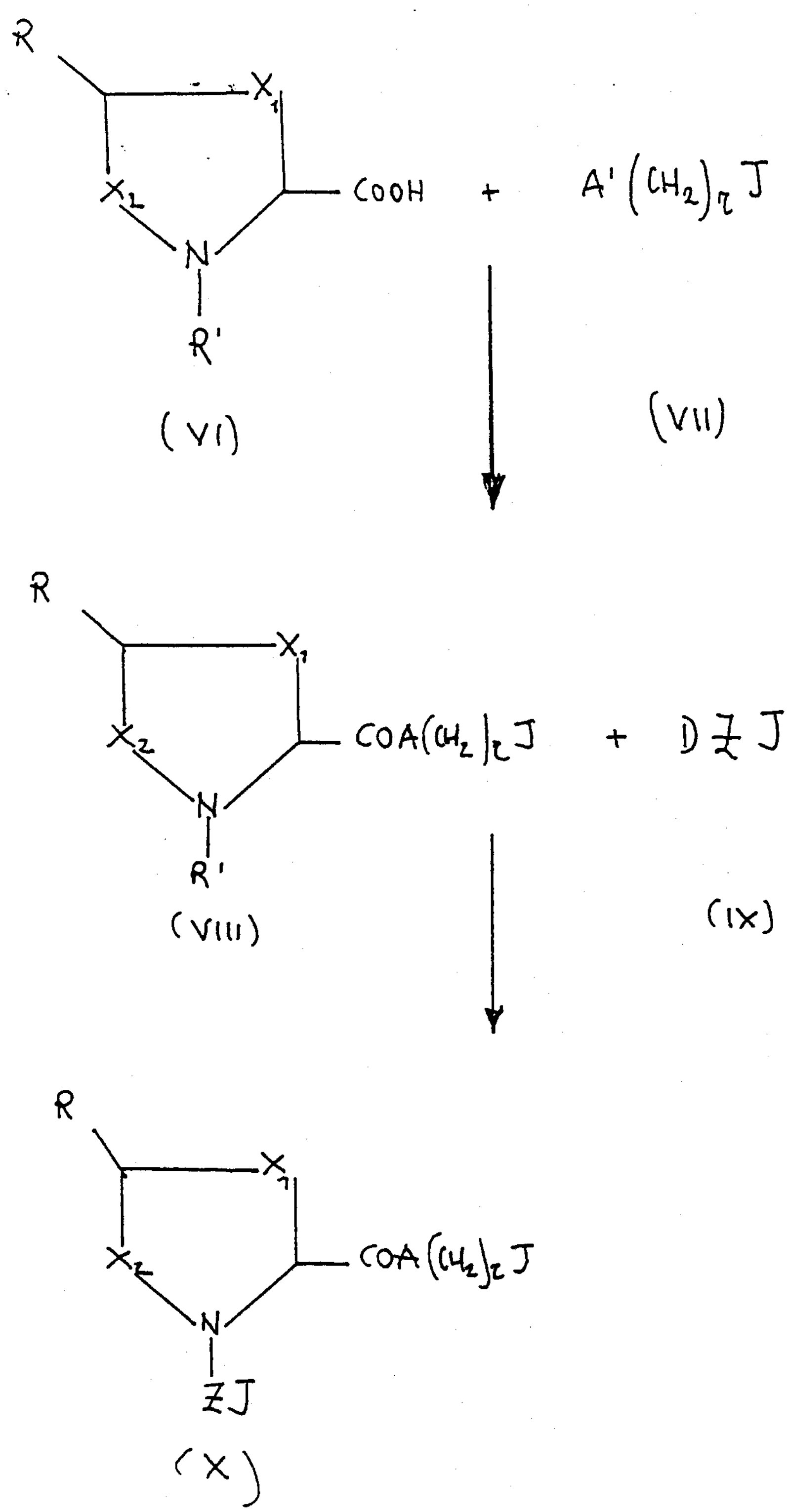

(VI) (VII) (VIII) (IX) (X)

Starting from general pattern G, the three types of compound I, II and III can be obtained according to the particular synthetic patterns A, B, C and D.

Synthetic pattern a) for obtaining compounds of formula I in which $X_1 = CH_2$.

In a first reaction step a compound of formula XII (corresponding to general formula VI in which $X_1 = X_2 = CH_2$) is reacted in the presence of carbonyldiimidazole with a compound of general formula XIII (corresponding to formula VII of the general pattern G in which A' = NHBz and J = $CH_3$; $0 \leq r \leq 6$; Bz = benzyl).

The resulting compound of formula XIV (corresponding to the compound of general formula VIII in which A = NBz), after removal of the protector group from the pyrrolidinic nitrogen by treatment with trifluoroacetic acid, is reacted with a compound of formula XVI (corresponding to general formula IX in which D is a leaning group, $Z=(CH_2)_p$ with p between 0 and 5 and J=COOBz). From this reaction the compound of general formula XVII is obtained, which, after catalytic hydrogenation on palladium to remove the protector groups to the carboxyl group anf to the nitrogen atom, gives the compound of formula XVIII in which $0 \leq p \leq 7$, which corresponds to a compound of general formula I in which $X_1 = CH_2$ and R = H, OH and in which $r=m+2$ and $p=m+2$.

The reaction diagram is shown below:

DIAGRAM a

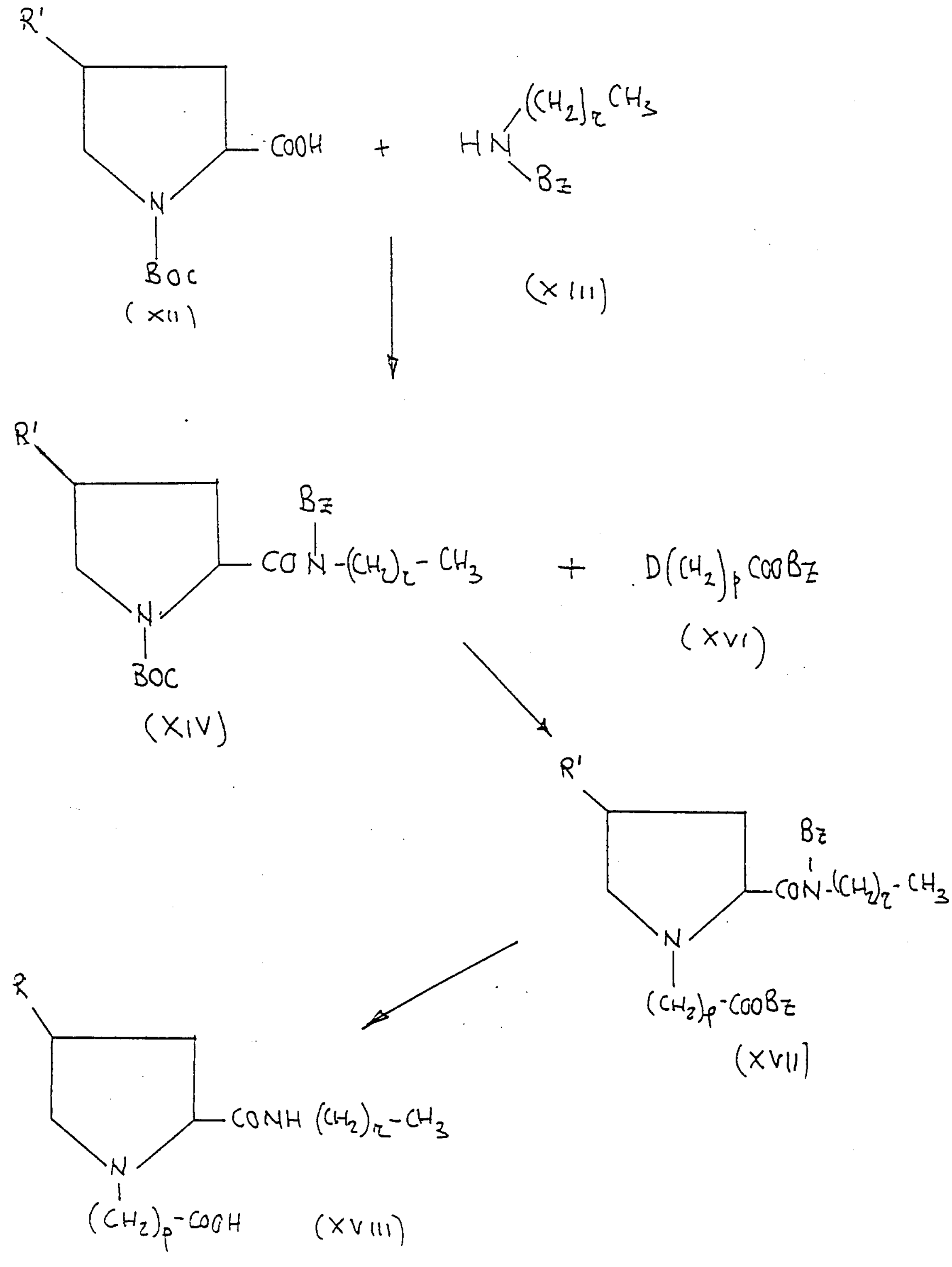

(XII) (XIII) (XIV) (XVI) (XVII) (XVIII)

Synthetic pattern b) for obtaining compounds of formula I in which $X_1$ = CO and R = H

A compound of formula XIX (corresponding to a compound of formula VI in which $X_2$ = CO, $X_1$ = $CH_2$, R = R' = H) is reacted in the presence of carbonyldiimidazole (CDI) with a compound of general formula XX (corresponding to a compound of formula VII in the general pattern in which A' = NHBz and J = $CH_3$

$0 \leq r \leq 7$ and Bz = benzyl).

The reaction produces a compound of general formula XXI (corresponding to a compound of general formula VIII in the general pattern in which A = NBz and J = $CH_3$). This compound is reacted in the presence of a base with a compound of formula XXII (corresponding to a compound of general formula IX in which J = COOBz, Z = $(CH_2)_p$ with $0 \leq p \leq 7$). The reaction produces a compound of formula XXIII (corresponding to a compound of general formula X in which A = NBz) which, after catalytic hydrogenation on palladium, produces a compound of general formula XXIII bis, which corresponds to a compound of general formula I in which $X_1$ = CO, R = H with p = n + 2 and r = m + 2.

The reaction pattern is shown below:

DIAGRAM b

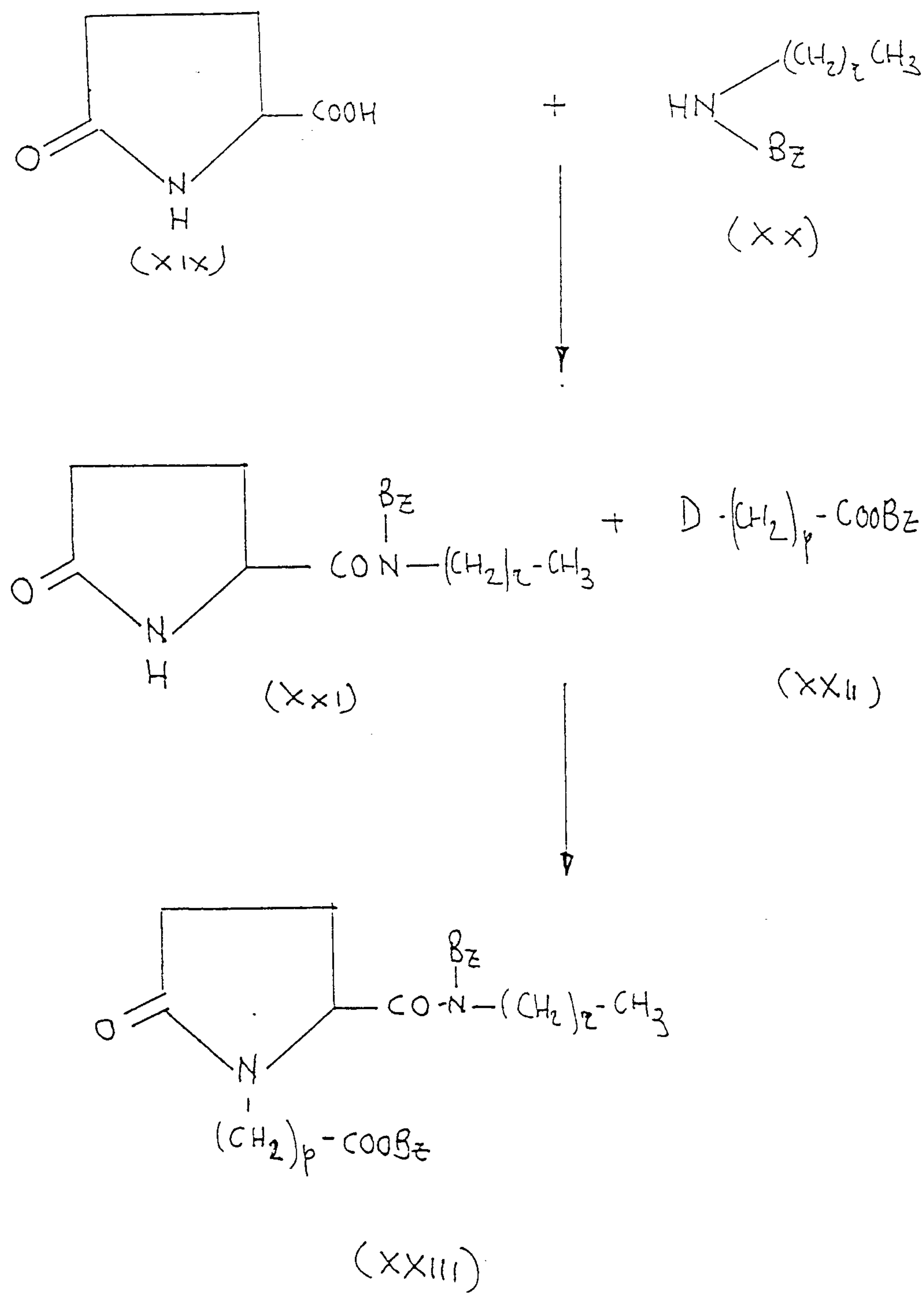

(XIX) (XX) (XXI) (XXII) (XXIII)

Synthetic patterns $c_1$, $c_2$ for obtaining compounds of formula II (A = NH) and III (A = O)

Pattern $C_1$ Diagram $c_1$ for obtaining compounds in which $X_2 = R_4 = CH_2$; $R_2 = R_6 = CO$.
Pattern $c_2$ Diagram $c_2$ for obtaining compounds in which $X_2 = R_2 = R_6 = CH_2$; $R_4 = CO$.

A compound of general formula XXIV (corresponding to the compound in general diagram VI in which $X_1 = X_2 = CH_2$) is reacted, respectively, with a compound of general formula XXV in the presence of dicyclohexylcarbodiimide according to pattern $c_1$ or with a compound of general formula XXVI in the presence of sodium hydroxide according to pattern $c_2$.

The compounds of formula XXV and XXVI correspond to the compounds of general formula VII in which A' is respectively the amino and halo group, $0 \leqq r \leqq 5$ and J = COOBz.

The compound of formula XXVII obtained from the reaction corresponds to a compound of general formula VIII in which A is NH in the case of pattern $c_1$, and is O in the case of pattern $c_2$.

After treatment with trifluoroacetic acid to remove protector group of the nitrogen atom in the pyrrolidinic ring, a compound of formula XXVIII is obtained, which is in turn reacted, in the presence of dicyclohexylcarbodiimide, with a compound of general formula XXIX corresponding to a compound of general formula IX in pattern G. The reaction product obtained, of formula XXXI, is then made to undergo catalytic hydrogenation, giving the compound of formula XXXIII, which corresponds to compounds of formula II in which A = NH or, respectively, of formula III in which A = O and in which $X_2 = R_4 = CH_2$ and $R_2 = R_6 = CO$. R = H, OH; r = n and p = m. The same compound of general formula XXVIII is reacted with the compound of general formula XXX (corresponding to the general formula IX in pattern G) to obtain the compound of general formula XXXII, which is made to undergo catalytic hydrogenation on palladium, giving the compound of general formula XXXIV which gives compounds of general formula II when A = NH and compounds of general formula III when A = O, in which $X_2 = R_2 = R_6 = CH_2$ and $R_4 = CO$; R = H, OH; r = n and p = m.

The reaction diagram is shown below:

DIAGRAM c

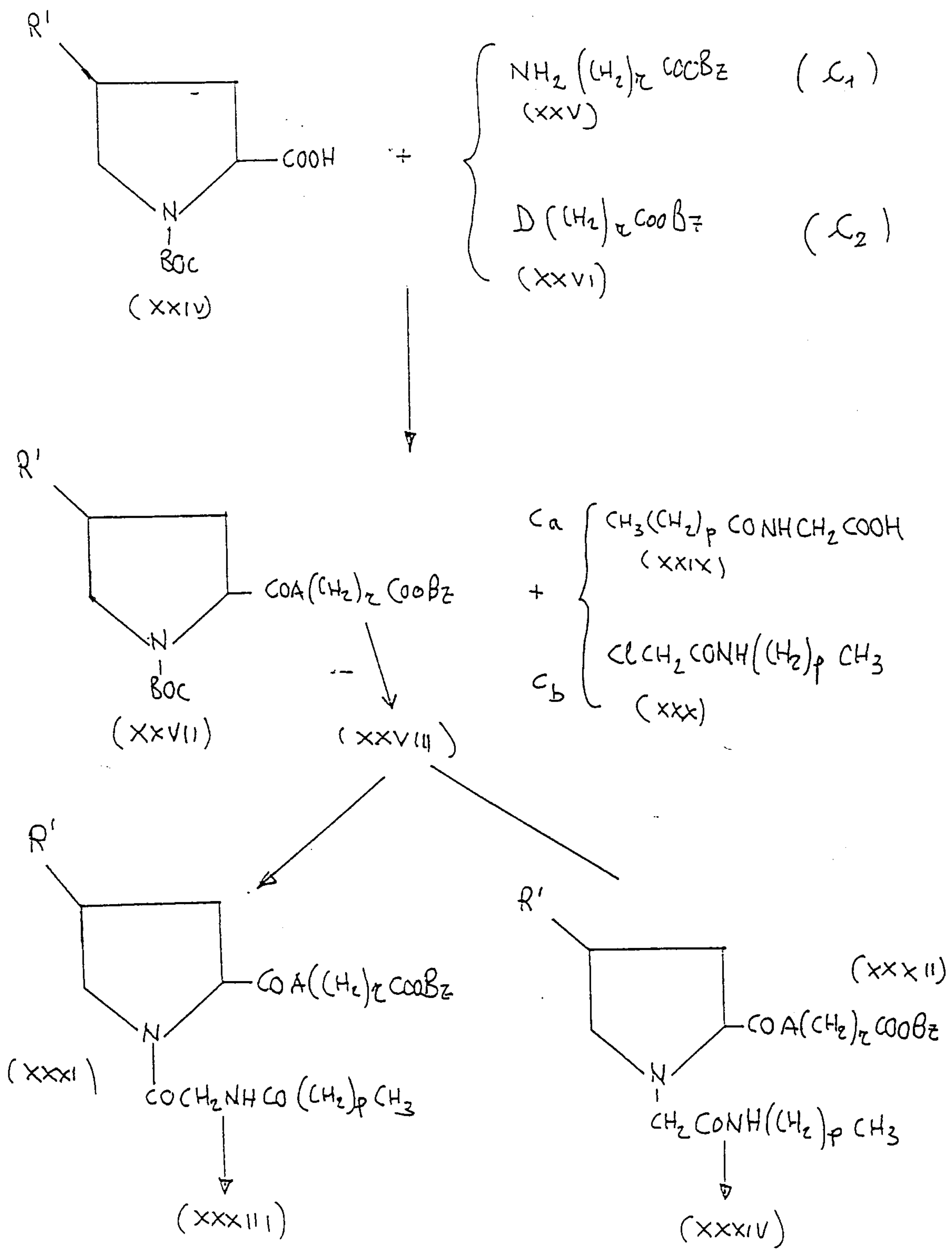


<u>Synthetic patterns $d_1$ and $d_2$ for obtaining compounds of general formula II in which A = NH and III in which A = O in which $X_2$ = $R_4$ = CO; R = H; $R_2$ = $R_6$ = $CH_2$.</u>

A compound of general formula XIX (corresponding to general formula VI in which $X_2$ = CO, $X_1$ = $CH_2$ and R = R' = H) is reacted in the presence of dicyclohexyilcarbodiimide with a compound of general

formula XXV according to the pattern $d_1$, in which $0 \leq r \leq 7$, (the other values are those indicated in patterns $c_1$ and $c_2$) and in the presence of sodium hydroxide with a compound of formula XXVI according to pattern $d_2$ (in this case also the other values are those shown in patterns $c_1$ and $c_2$). The compound obtained, of formula XXXI, corresponds to the compound of general formula VIII in which A = NH in the case of pattern $d_1$, and A = O in the case of pattern $d_2$. The compound of formula XXV is reacted, in the presence of a base, with a compound of formula XXXVI, which corresponds to the compound of general formula IX in pattern G $= \leq p \leq 5$). The reaction product, of formula XXXVII, is then made to undergo catalytic hydrogenation on palladium to remove the protector groups, giving compounds of formula II when A = NH, or of formula III when A = O, in which $X_2 = R_4 = CO$; R = H; $R_2 = R_6 = CH_2$, r = n and m = p.

**DIAGRAM d**

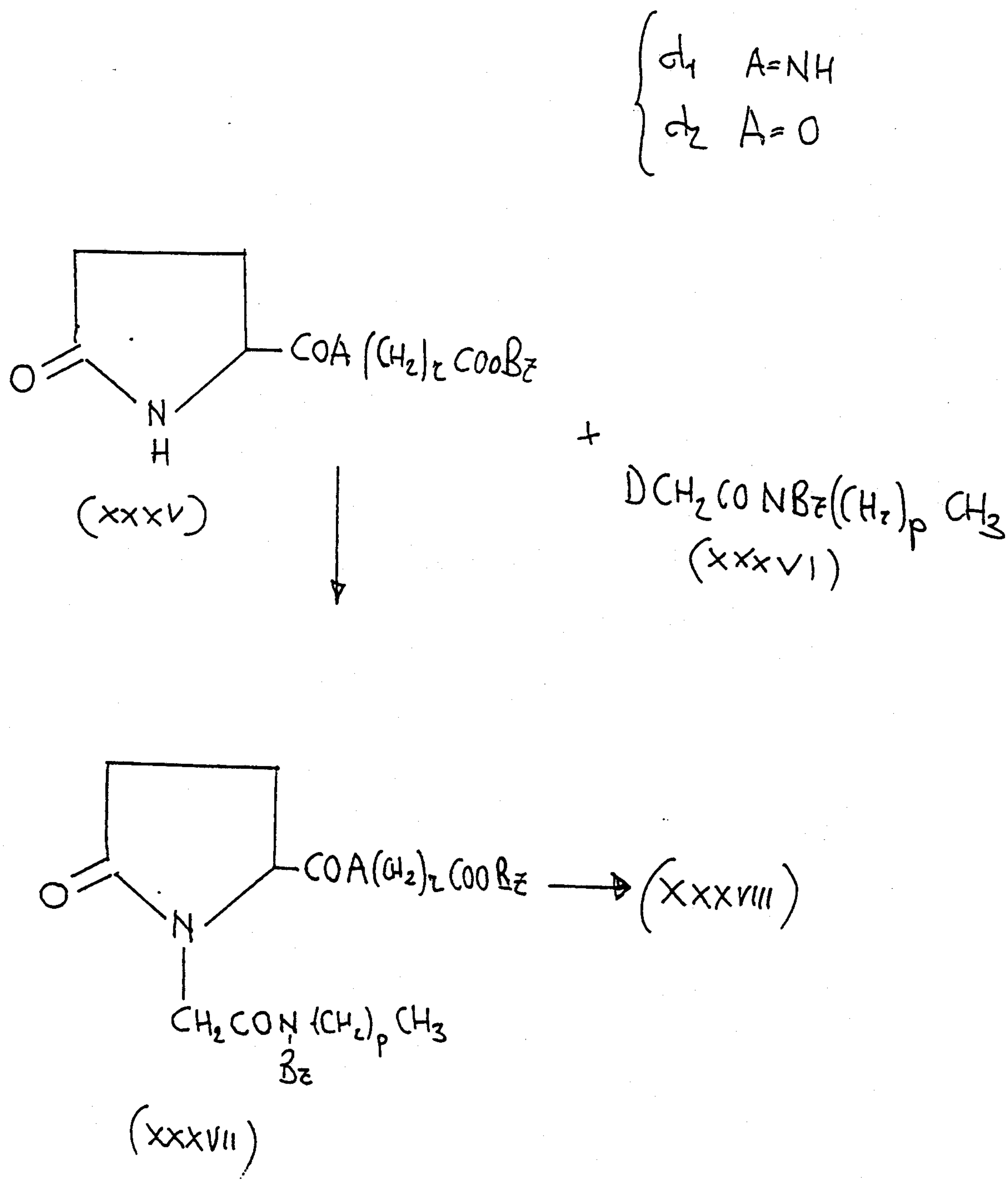


(XXXV)

(XXXVI)

(XXXVIII)

(XXXVII)

The synthetic patterns given above require the introduction into at least one of the two chains of the heterocycloaliphatic ring of at least one carboxamidic group. In the following several examples of preparation of the compounds according to the present invention are shown, which in no way are to be intended as limiting the scope of said invention. In all preparations, both the single intermediates and the final products have been analyzed by elementary microanalysis, IR spectroscopy, 1H- and 13C-RMN spectroscopy, which have confirmed the structural formulae expected.

Reactions were performed using thin layer chromatography (TLC) on fluorescent silica gel plate with UV or iodine vapor detection until disappearance of one of the starting materials. The solvents used to perform the reactions are anhydrous. In the absence of indications to the contrary, the reaction mixture was worked by dividing between water and chloroform, washing the chloroform phase repeatedly with water to remove any hydro-soluble solvents (for example dimethylsulphoxide DMSO) and if necessary with aqueous sodium bicarbonate and/or chlorhydric acid to remove any residual starting materials or impurities of an acid or basic nature; the organic phase was dehydrated using anhydrous sodium sulphate or anhydrous potassium carbonate in the case of basic products.

Preparative chromatographic separations were performed on silica gel column monitoring the process by means of thin layer chromatography. The eluents used for preparative or thin layer chromatography (TLC) are the following (for which the RF values are given)

a) Hex + AcOE 7:3
b) Hex + AcOE + MeOH 5:4:1
c) AcOE + AcOH 9:1
d) Hex + AcOE + AcOH 5:4:1

In the following text, the following abbreviations have been used: AcOE = ethyl acetate; AcOH = acetic acid; CDI = carbonyldiimidazole; DCC = dicyclohexylcarbodiimide; DCU = dicyclohexylurea; DMSO = dimethylsulphoxide; HeX = n-hexane; HoBT = 1-hydroxybenzotriazole; MeOH = methanol; Pd/C = palladium on 10% carbon; TFA = trifluoroacetic acid; THF = tetrahydrofurane; BOC = terzbutoxycarbonyl; Bz = benzyl; r.t. = room temperature; a.p. = atmospheric pressure; u.m.s = under magnetic stirring.

Example 1

Synthesis of L-9,13-dioxo-8,14-diazaprostanoic acid (according to synthetic pattern b).

Compound of formula I with $X_1$ = CO, R = H, n = 4, m = 3.

Equimolecular amounts of benzaldehyde and n-hexylamine are intimately mixed; as heat develops the mixture separates into two phases. The lower aqueous phase is separated from the organic phase, which is dissolved in an organic solvent and dried using anhydrous potassium carbonate; evaporation of the solvent leaves an oily residue of n-hexyl-benzaldimine (yield 87%). 13 g of this intermediate, dissolved in 100 ml of ethanol and additioned with 8 ml of ethanolic suspension of Raney nickel, are hydrogenated at r.t. and 7 atm. for 20 hours. The solution filtered off from the catalyst is dry evaporated and the residue is distilled under vacuum to give 78% yield of benzyl-hexyl-amine b.p. 150° (12 mm Hg). Purification of this amine is performed by transforming it into a chlorhydrate by addition of ethanolic chlorhydric acid to its ether solution: b.p. 112°C.

20 mmoles of L-pyroglutamic acid (formula XIX) are set to react in 60 ml of THF + 60 ml of DMSO with 20 mmoles of CDI until effervescence ceases. 10 mmoles of pyridine and 10 mmoles of benzylhexylamine (corresponding to a compound of formula XX) are then added and the mixture is kept under magnetic stirring for one night and then refluxed for 20 hours until the reaction is complete. The product obtained after working of the mixture is an oil (89% raw yield) which is undergone to cromatography collecting the fraction with RF = 0.75 (eluent C) yield 72%, made of the benzylhexylamide of the pyroglutamic acid (compound XXI; R = 5).

One mmole of this intemediate dissolved in 20 ml of DMSO is added to 30 ml of t-butanol containing 1 mmole of sodium terz-butoxide.

1 mmole of benzyl 7-bromoheptanoate (formula XXI p = 6), keeping the mixture under magnetic stirring for one night and then refluxing 20 hours until the reaction is completed, is then added.

The reaction product benzylester of L-14-benzyl-9,13-dioxo-8,14-diazaprostanoic acid (compound XXIII r = 5 p = 6) is isolated by cromatography RF = 0.8 (eluent C) yield 73%.

5 moles of the intermediate just obtained dissolved in 30 ml of dioxane and 3.5 ml of AcOH are hydrogenated in the presence of 0.3 g of Pd/C under r.t. and a.p. for three days.

The solution filtered of from the catalyst and evaporated gives L-9,13-dioxo-8,14-diazaprostanoic acid as oily liquid RF 0.4 (eluent C) $[\alpha]_D$ = -2° with a yield of 97%.

Example 2.

Synthesis of L-10-hydroxy-7,13,16-trioxo-6,12,15-triazaprostanoic acid (synthetic pattern $c_1$).

Compound of formula II with $X_2$ and $R_4$ = $CH_2$ and $R_2$ and $R_6$ = CO R = OH n = 4 m = 3.

50 mmoles of 4-benzyloxy-N-BOC-L-proline (corresponding to the general formula XXIV) in 120 ml of dioxane + 5 ml of pyridine + 8 ml of trietilamine are reacted with 53 mmoles of benzyl 5-amino-pentanoate chlorhydrate (formula XXV r = 4) in the presence of equimolar amounts of DCC and HOBt at r.t. for three days.

The separated DCU is filtered off and the mixture worked to give a cristalline residue of benzyl 5-(L-N-BOC-4-benzyloxyprolilamino)-pentanoate (formula XXVII R' = OBz A = NH r = 4). m.p. 52 ° C RF = 0.6 (eluent B) yield 82%.

50 mmoles of the intermediate just obtained are dissolved in 70 ml of chloroform + 70 ml of TFA and the mixture kept under magnetic stirring at r.t. for two hours.

The mixture is evaporated, neutralized with aqueous sodium bicarbonate and extracted with chloroform to give, after evaporation, an oily residue of benzyl 5-(L-4-benzyloxyprolilamino)-pentanoate in almost theoretical yield RF = 0.4 (eluent C).

10 mmoles of the just obtained intermediate (formula not shown in pattern C) dissolved in 60 ml of dioxane and 1 ml of pyridine are reacted with 12 mmoles of N-pentanoyl-glycine (formula XXIX p = 3) in the presence of equimolar amounts of DCC + HOBt for four days at r.t.

The DCU separated is filtered and worked to obtain an oily liquid made of benzyl 5-(N-pentanoylglycil-4-benzyloxy-L-prolilamino)-pentanoate (formula XXI) RF = 0.8 yield 72%.

5 mmoles of the last intemediate dissolved in 27 ml of dioxane + 3 ml of AcOH are hydrogenated in the AcOH presence of 0.3 g of Pd/C at r.t. and atmospheric pressure for three days.

The solution is filtered and separated from the solvents to give L-10-hydroxy-7,13,16-trioxo-6,12,15-triazaprostanoic acid m.p. 78 ° C; RF = 0.3 (eluent C) $[\alpha]_D$ = -62 ° yield 98%.

Example 3.

Synthesis of the L-7-14-dioxo-12,15-diaza-6-oxa-prostanoic acid (synthetic pattern $C_2$) (formula III $X_2 = R_2 = R_6 = CH_2$ $R_4$ = CO R = H m = 3 n = 4)

To 15 mmoles of sodium hydroxide dissolved in 10 ml of anhydrons ethanole diluted with 22 ml of DMSO, are added with 19 mmoles of N-BOC-L-proline (formula XXIV) and anhydrons ethanol until the formed precipitate is dissolved.

12 mmoles of benzyl 5-bromopentanoate (formula XXVI R = 4) are then added and mixture is refluxed for twenty hours until the reaction is completed.

The working gives an oily residue (yield 93%) made of benzyl 5-(N-BOC-L-proliloxypentanoate) (compound XXVII R' = H A = O) sufficiently pure for the next step.

11 mmoles of the intermediate just obtained are treated with 12 ml of chloroform and 12 ml of TFA at r.t. for two hours to give, after evaporation of the solvents, a syropy residue made of trifluoroacetate of benzyl 5-(L-proliloxy)-pentanoate (XXVIII).

This compound is transformed into the free base by a treatment at 0 ° C in 40 ml of chloroform with 90 ml of an ice-aqueous solution of sodium carbonate at 5% in five portions.

The organic phase is dryed on anhydrons potassium carbonate at 0 ° C for twentyfour hours.

The phasis then evaporated to dryness under vacuum at low temperature and free base is immediately dissolved in 12 ml of DMSO and stirred at r.t. with 6 mmoles of N-pentyl-chloroacetamide (formula XXX p = 4) and 6 mmoles of triethylamine for one night.

The working of the mixture gives, after chromatographic separation, an oily fraction RF = 0.8 (eluent C) made of acetate of benzyl 5-(N-pentylaminocarbonylmethyl-L-proliloxy)-pentanoate (formula XXXII R = H A = O r = 4 p = 4) with yield of 67%.

3.9 mmoles of the just obtained compound and 27 ml of dioxane + 3 ml of AcOH are hydrogenated in the presence of 0.26 g of Pd/C at r.t. and a.p. for three days to give, after filtration and evaporation, the L-7,14-dioxo-12,15-diaza-6-oxaprostanoic acid acetate as oily liquid RF = 0.5 (eluent C) $[\alpha]_D$ = -66% with yield of 90%.

Example 4.

Synthesis of the L-7,13,16-trioxo-12,15-diaza-6-oxa-prostanoic (synthetic pattern $c_2$) (formula III with $X_2 = R_4 = CH_2$ $R_2 = R_6 = CO$ R' = H n = 4 m = 3)

6.5 mmoles of the compound XXVIII (R' = H A = O r = 4) of the preparation according to example 3 under the form of free base without trifluoroacetate, are dissolved in 5 ml of THF and added with 6.5 mmoles of pyridine.

A solution of 6.5 mmoles of N-pentanoylglycine (formula XXIX p = 4) in 25 ml of THF is prepared separately, which is then added with 13 mmoles of CDI.

After the effervescence is finished, to this solution is added the preceding solution and the reaction mixture is kept one night under magnetic stirring at r.t.

The working gives a raw oil (82% of yield) which is chromatographated to give a fraction with RF = 0.8 (eluent C) made of benzyl 5-(N-pentanoylglycil-L-proliloxy)-pentanoate (intermediate XXXI R' = H A = O r = 4 p = 4) with a yield of 75%.

5 mmoles of compound XXXI in 30 ml of dioxane + 3.5 ml of AcOH are hydrogenated in the presence of 0.3 g of Pd/C at r.t. and atmospheric pressure for three days to give, after filtration and evaporation of the solvents, L-7,13,16-trioxo-12,15-diaza-6-oxa-prostanoic acid as oily liquid RF = 0.5 (eluent C) $[\alpha]_D = -78°$ with yield of 99%.

Example 5.

Synthesis of the L-7,11,14-trioxo-12,15-diaza-6-oxa-prostanoic (synthetic pattern $d_2$) (formula III with $X_2 = R_4 = CO$; $R_2 = R_6 = CH_2$ R = H n = 4 m = 3).

17 mmoles of pyroglutamic acid (formula XIX) are dissolved in solution of 16 mmoles of sodium hydroxid in 15 ml of ethanol.

The solution is diluted with 37 ml of DMSO and 12 mmoles of benzyl 5-bromopentanoate (formula XXVI r = 4) are added.

The mixture is left under magnetic stirring at r.t. for twentyfour hours and then is worked to give an oil made of benzyl 5-L-pyroglutamiloxy pentanoate (compound XXXV A = O r = 4) RF 0.8 (eluent B) sufficiently pure for following step (yield 88%).

14 mmoles of N-pentylbenzylamine chloridrate suspended in 18 ml of dichloroethane are added to 10 ml of aqueous sodium hydroxide at 20% and treated at 0 °C under magnetic strirring with 40 mmoles of chloroacetyl chloride added dropwise.

The organic phase free from acids and bases and dryed is then evaporated to give N-benzyl-N-pentyl-chloroacetamide as pure liquid (RF 0.7) (eluent A) with yield 86%.

12 mmoles of the compound just obtained in 5 ml of acetone are treated with 12 mmoles of sodium iodide to give, after filtration of sodium chloride which has separated and evaporation of the solvent, N-benzyl-N-pentyl-iodoacetamide (formula XXXVI D = I) as oily liquid RF = 0.7 (eluent A) yield 99%.

3 mmoles of the compound XXXV obtained above, dissolved in 35 ml of terz-butanol containing 3 mmoles of sodium t-butoxide, are left under magnetic stirring for one night.

The formed precipitate is dissolved with 20 ml of DMSO and the mixture added with 3 mmoles of the compound XXXVI and is kept under reflux 48 hours until the reaction is completed.

The reaction mixture worked gives, after chromatography, an oily fraction RF = 0.8 (eluent D) made of benzyl 5-(N-benzylpentylaminocarbonylmethyl-L-pyroglutamiloxy)-pentanoate (compound XXXVII A = O r = p = 4) with yield of 85%.

10 mmoles of the compound just obtained dissolved in 60 ml of dioxane and 7 ml of AcOH are hydrogenated in the presence of 0.6 g of Pd/C at r.t. and a.p. for three days to give, after filtration and evaporation, L-7,11,14-trioxo-12,15-diaza-6-oxaprostanoic acid as oily liquid; RF = 0.5 (eluent C) $[\alpha]_D = +2°$ yield = 98%.

PHARMACOLOGICAL ASSAYS

The therapeutical activities of the compounds according to the present invention have been compared with some prostaglandins of natural origin.

a) Activity on the smooth muscles

Some compounds according to the present invention have been tested and found active in vitro on ilium of guinea-pig in Tyrode solution oxygenated to which some receptorial antagonists in suitable concentrations have been added (colinergic, adrenergic, histaminic and seratoninic).

The results of these pharmacological assays concerning the activity on the smooth muscles are presented under the form of table (table I), the indicated value being the equiactive concentration with respect to the compound $PGE_2$ provoking a submaximal contraction of the preparation.

TABLE I

| Compound | Concentration (M) |
|---|---|
| $PGE_2$ | $5.10^{-7}$ |
| I ($X_1$ = CO, R = H, n = 4, m = 4) | $2.10^{-4}$ |
| II ($X_2$ = $R_4$ = $CH_2$, $R_2$ = $R_6$ = CO, R = H, n = 4, m = 3) | $5.10^{-4}$ |
| III ($X_2$ $R_4$ = $CH_2$, $R_2$ = $R_6$ = CO, R = H, n = 4, m = 3) | $5.10^{-4}$ |
| III ($X_2$ = $R_2$ = $R_6$ = $CH_2$, $R_4$ = CO, R = H, n = 4, m = 3) | $1.10^{-4}$ |
| III ($X_2$ = $R_2$ = $R_6$ = $CH_2$, $R_4$ = CO, R = OH, n = 4, m = 3) | $3.10^{-5}$ |
| III ($X_2$ $R_4$ = CO, $R_2$ $R_6$ = $CH_2$, R = H, n = 4, m = 3) | $8.10^{-5}$ |

b) Activity on the thrombocyte aggregation

Some of the compounds described in the present invention have been tested and found active as inhibitors of the thrombocyte aggregation in comparison to $PGE_1$.

The assey has been carried out on the plasma of a rat rich in thrombocytes obtained by centrifuging the blood added with cytrate at 250 g.

The reversible trombocyte aggregation has been obtained by adding ADP in concentration 6.3 $10^{-6}$ M and measured by the maximum decrease of absorbance which takes place after about 1 minute.

The compounds to be tested and the PG used as a comparison dissolved in tris buffer at three different concentrations, are added to the plasma one minute before the addition of ADP.

The $EC_{50}$s for the different compounds can be obtained from the measured values and are listed in Table II.

The $EC_{50}$s are defined as the molar concentrations which inhibit for the 50% the aggregation induced by ADP.

TABLE II

| Compound | $EC_{50}$ (M) |
|---|---|
| $PGE_1$ | $1,10^{-7}$ |
| I ($X_1$ = C0, R = H, n = 4, m = 4) | $3,10^{-3}$ |
| II ($X_2$ = $R_4$ = $CH_2$, $R_2$ = $R_6$ = CO, R = H, n = 4, m = 3) | $1,1,10^{-3}$ |
| III ($X_2$ = $R_4$ = $CH_2$, $R_2$ = $R_6$ = CO, R = H, n = 4, m = 3) | $6,10^{-4}$ |
| III ($X_2$ = $R_2$ = $R_6$ = $CH_2$, $R_4$ = CO, R = H, n = 4, m = 3) | $5,10^{-4}$ |
| III ($X_2$ = $R_2$ = $R_6$ = $CH_2$, $R_4$ = CO, R = OH, n = 4, m = 3) | $3,10^{-5}$ |
| III ($X_2$ = $R_4$ = CO, $R_2$ = $R_6$ = $CH_2$, R = H, n = 4, m = 3) | $5,10^{-6}$ |

STABILITY ASSAYS

The stability of some compounds according to the present invention has been compared with that of a natural prostaglandine through storage at room temperature of water solutions buffered at pH 7, by measuring the residual activity in the assay of thrombocyte aggregation at different times; the results, listed in Table III, show a much greater stability:

TABLE III

| Compound | 1h | 12h | 24h | 7 gg |
|---|---|---|---|---|
| $PGE_1$ | 30 | 2 | 0 | 0 |
| II ($X_2=R_4=CH_2$, $R_2=R_6=CO$, R=H, n=4, m=3) | 100 | 98 | 96 | 40 |
| III ($X_2=R_4=CH_2$, $R_2=R_6=CO$, R=H, n=4, m=3) | 100 | 92 | 80 | 15 |
| III ( $X_2=R_2=R_6=CH_2$, $R_4=CO$, R=H, n=4, m=3) | 98 | 78 | 62 | 4 |

EXAMPLES OF PHARMACEUTICAL COMPOSITIONS

a) Parenteral administration

The compounds of the present invention may be used as injectable preparations at a dosage of 10-100 mg both in oily vehicle and in buffered water vehicle in which the active principle is solubilized when used.

b) Oral administration

The compounds according to the present invention may be administered orally in gelatin capsules, mixed with vehicles or excipient tolerable with oral administration; dosage 20-500 mg.

c) Rectal administration

The compounds according to the present invention may be used together with a normal excipient for suppositories for the rectal administration at dosage of 50-500 mg.

## Claims

**1.** Compounds of general formula IV

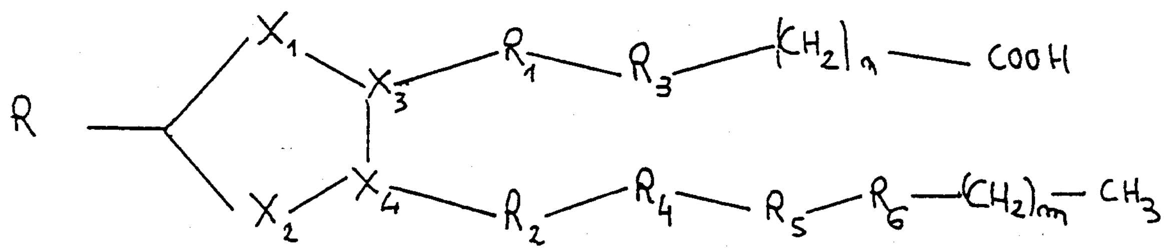

in which is $X_1$-$CH_2$, CO; $X_2 = CH_2$, CO; $X_3 = N$, CH; $X_4 = N$, CH; R = H, OH; $R_1 = CH_2$, CO; $R_2 = CO$,

$CH_2$; $R_3 = CH_2$, NH, O; $R_4 = NH$, $CH_2$, CO; $R_5 = CH_2$, NH; $R_6 = CH_2$, CO; m is between 0 and 4, n is between 0 and 5.

and pharmaceutically tolerable salts thereof.

**2.** Compounds according to claim 1 of general formula I

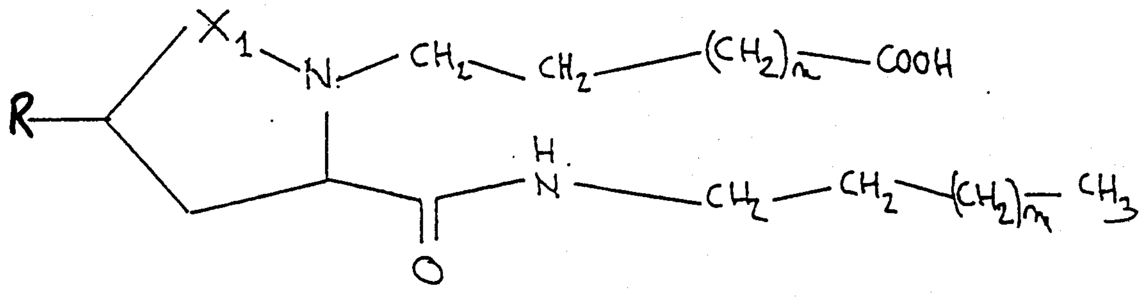

corresponding to general formula IV,

in which $X_2 = CH_2$; $X_3 = N$; $X_4 = CH$; $R_1 = CH_2$; $R_2 = CO$; $R_3 = CH_2$; $R_4 = NH$; $R_5 = CH_2$; $R_6 = CH_2$, and pharmaceutically tolerable salts thereof.

**3.** Compounds according to claim 1 of formula II

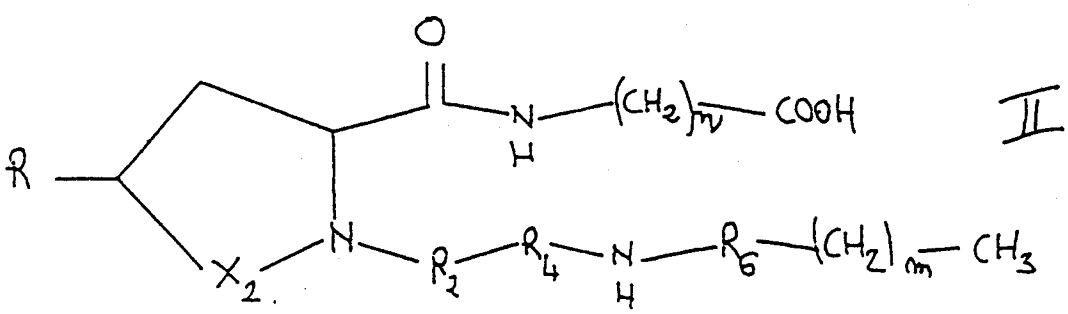

corresponding to general formula IV,

in which $X_1 = CH_2$; $X_3 = CH$; $X_4 = N$; $R_1 = CO$; $R_3 = NH$; $R_5 = NH$;

and pharmaceutically tolerable salts thereof.

**4.** Compounds according to claim 1 of general formula III

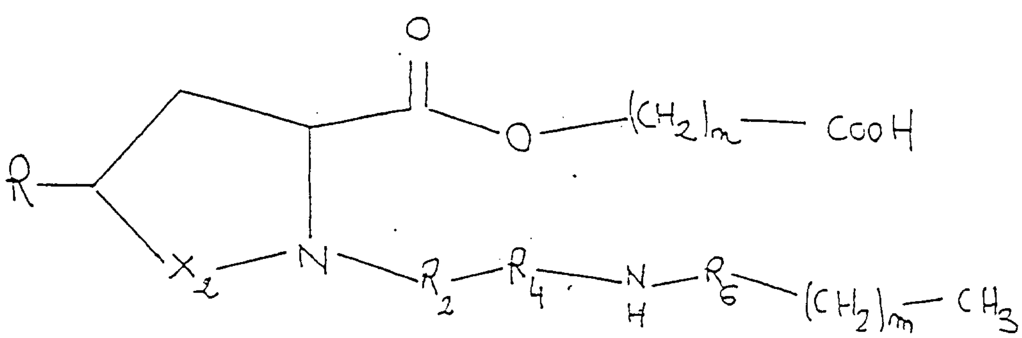

corresponding to general formula IV,

in which $X_1 = CH_2$; $X_3 = CH$; $X_4 = N$; $R_1 = CO$; $R_3 = O$; $R_5 = NH$; and pharmaceutically tolerable salts thereof.

**5.** Process for the production of compounds of general formula IV comprising the following steps:

1) transformation of a carboxylic compound of general formula VI, in which R = H, OBz and R' = H, BOC into a functional derivative thereof to the carboxylic function of formula VIII, by reacting it with a compound of general formula VII in which A' = halogen, NHBz or $NH_2$, J = $CH_3$, COOH, the COOK group being optionally protected, and with $0 \leqq r \leqq 7$;
2) reacting the compound of general formula VIII in which A = O, NH or NBz, if necessary after deprotection of the R' group, with a compound of formula IX in which D is an halogen or OH, and Z is a methylenic chain with up to 8 members, optionally interrupted by CO and/or NH groups, to give the compound of general formula X; it being possible to invert the order of operations 1 and 2; and
3) catalytic hydrogenation of the compound of general formula X so as to free any protected functions and to give the compounds of formula XI which can be brought back to compounds of general formula IV.

**6.** Use of a compound as claimed in claims 1 to 4 as an active agent on smooth muscle.

**7.** Use of a compound according to claims 1 to 4 as an active agent against thrombocyte aggregation.

**8.** A pharmaceutical composition comprising a therapeutically effective amount of a compound as claimed in claims 1 to 4 together with a pharmaceutically tolerable vehicle.